(19) Europäisches Patentamt / European Patent Office / Office européen des brevets

(11) **EP 2 342 354 B1**

(12) **EUROPEAN PATENT SPECIFICATION**

(45) Date of publication and mention of the grant of the patent:
**11.02.2015 Bulletin 2015/07**

(21) Application number: **09744450.9**

(22) Date of filing: **09.09.2009**

(51) Int Cl.:
*C12Q 1/68* (2006.01)     *G01N 33/574* (2006.01)

(86) International application number:
**PCT/IB2009/007099**

(87) International publication number:
**WO 2010/029440 (18.03.2010 Gazette 2010/11)**

(54) **MOLECULAR CLASSIFIER FOR EVALUATING THE RISK OF METASTASIC RELAPSE IN BREAST CANCER**

MOLEKULARKLASSIFIZIERUNG ZUR BEWERTUNG DES RISIKOS VON METASTASENRÜCKFÄLLEN BEI BRUSTKREBS

Classificateur moléculaire pour évaluer le risque de rechute métastasique dans le cancer du sein

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **11.09.2008 EP 08290854**

(43) Date of publication of application:
**13.07.2011 Bulletin 2011/28**

(73) Proprietors:
- **Fédération Nationale des Centres de Lutte Contre le Cancer**
  **75013 Paris (FR)**
- **Centre de Lutte contre le Cancer René Gauducheau**
  **44805 Saint Herblain (FR)**
- **Hôtel-Dieu**
  **44093 Nantes Cedex 1 (FR)**

(72) Inventors:
- **JEZEQUEL, Pascal**
  **F-44805 Saint Herblain (FR)**
- **CAMPION, Loïc**
  **F-44805 Saint Herblain (FR)**
- **MINVIELLE, Stéphane**
  **F-44007 Nantes Cedex 1 (FR)**

(74) Representative: **Leblois-Préhaud, Hélène Marthe Georgette et al**
  **Cabinet Orès**
  **36, rue de St Pétersbourg**
  **75008 Paris (FR)**

(56) References cited:
- **MARIO CAMPONE ET AL: "Prediction of metastatic relapse in node-positive breast cancer: establishment of a clinicogenomic model after FEC100 adjuvant regimen" BREAST CANCER RESEARCH AND TREATMENT, KLUWER ACADEMIC PUBLISHERS, BO, vol. 109, no. 3, 21 July 2007 (2007-07-21), pages 491-501, XP019600649 ISSN: 1573-7217**
- **SOTIRIOU CHRISTOS ET AL: "Gene expression profiling in breast cancer: understanding the molecular basis of histologic grade to improve prognosis" NATIONAL CANCER INSTITUTE. JOURNAL (ONLINE), OXFORD UNIVERSITY PRESS, GB, vol. 98, no. 4, 15 February 2006 (2006-02-15), pages 262-272, XP002490627 ISSN: 1460-2105**
- **WILSON C L ET AL: "Effects of oestrogen on gene expression in epithelium and stroma of normal human breast tissue" ENDOCRINE-RELATED CANCER, vol. 13, no. 2, June 2006 (2006-06), pages 617-628, XP002518294 ISSN: 1351-0088**
- **TURNER DAVID P ET AL: "Global gene expression analysis identifies PDEF transcriptional networks regulating cell migration during cancer progression" MOLECULAR BIOLOGY OF THE CELL, vol. 19, no. 9, September 2008 (2008-09), pages 3745-3757, XP002518295 ISSN: 1059-1524**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **VAN'T VEER L J ET AL: "Gene expression profiling predicts clinical outcome of breast cancer" NATURE, NATURE PUBLISHING GROUP, LONDON, UK, vol. 415, 31 January 2002 (2002-01-31), pages 530-535, XP003020164 ISSN: 0028-0836**

**Description**

The invention relates to tools and methods for predicting evolution in breast cancer.

Primary therapy for early stage breast cancer generally involves local treatment by surgery and/or radiation therapy. However, micrometastases that are clinically undetectable may escape the treatment, leading to reappearance of the disease.

The use of adjuvant systemic therapy, i.e. systemic anticancer therapy given in addition to the primary treatment, aims at eliminating these micrometastases in order to prevent or delay relapses and increase the chance of long-term survival.

Adjuvant chemotherapy involves the use of anticancer drugs, generally in combination. The most frequently used include anthracyclines such as doxorubicin or epirubicin, 5-fluorouracil, methotrexate, cyclophosphamide, and taxoids such as paclitaxel or docetaxel. Adjuvant hormone therapy uses estrogen analogues such as tamoxifen, that reduce the growth of cancer cells by blocking the estrogen receptor (ER).

Adjuvant therapy is often associated with various adverse effects such as alopecia, nausea and vomiting. Although having fewer side effects than chemotherapy, tamoxifen has been reported to cause venous thromboembolism or endometrial cancer.

Not all patients will derive benefit from adjuvant therapy. For instance, in patients having a good prognosis after primary therapy the disease is less likely to recur and thus these patients are less likely to obtain an additional benefit from adjuvant systemic therapy. Further, among the patients who are eligible for adjuvant therapy, the response to a given type of therapy may vary from an individual to another.

It is thus important to identify factors associated with prognosis, which reflect the aggressiveness of a tumor, and are useful to estimate the outcome of the disease independently of the treatment administered, and also factors allowing to predict the sensitivity or resistance of a tumor to a given therapeutic agent. This prognostic and predictive factors are respectively useful to evaluate whether a patient is likely to benefit from adjuvant therapy and which kind of therapy is most likely to work for this patient.

Several factors correlated with prognosis, some of which have also a predictive value, have been identified. They include in particular tumor size, lymph node status, histological grade, proliferative capacity of the tumor, expression of ER, and expression of the oncogene HER-2/neu (erbB-2). Various combinations of these factors have been integrated into multivariate models allowing to estimate risks for recurrence and mortality and to estimate the potential benefits of adjuvant therapy. Among the more widely used models one can cite the Nottingham Prognostic Index (GALEA et al. Breast Cancer Res Treat, 22, 207-219, 1992), which is based on tumor size, lymph node stage and histological grade, and the Adjuvant! Computer program (RAVDIN et al., J Clin Oncol, 19, 980-991, 2001), which provides prognostic estimates from patient information (age, menopausal status, comorbidity) and tumor factors (tumor size, lymph node status, ER status).

More recently, it has been proposed to use gene expression profiling first for identifying gene-expression signatures (GES) distinguishing breast tumors into subclasses with clinical implications, and second to predict evolution of node-negative breast cancer patients (PEROU et al., Nature, 406, 747-52, 2000; SORLIE et al. Proc Natl Acad Sci USA, 98, 10869-74, 2001; VAN'T VEER et al., Nature, 415, 530-35, 2002; VAN DE VIJVER et al., N Engl J Med, 347, 1999-2009, 2002; WANG et al., The Lancet, 365, 671-79, 2005)

The initial focus on node-negative breast cancer subtyping resulted from the crucial decision of not treating low-risk patients who might have a favorable evolution without systemic adjuvant chemotherapy. Currently, three commercially available prognostic breast cancer tests resulting from gene expression studies are available: Oncotype Dx(Genomic Health, Redwood City, CA, USA), MammaPrint(Agendia BV, Amsterdam, the Netherlands) and H/I (AvariaDX, Carlsbad, CA, USA) (PAIK et al., N Engl J Med, 351, 2817-26, 2004; MA et al., Cancer Cell, 5, 607- 16, 2004)

All these tests are designed to be used in the management of node-negative breast cancer. Today, no robust GES able to predict node-positive breast cancer evolution after systemic adjuvant chemotherapy exists. Being able to type those node-positive patients would lead to orientate and include high-risk patients in new clinical trials testing novel targeted therapies.

The goal of the inventors was thus to identify and validate a GES for the purpose of predicting metastatic relapse (MR) in node-positive patients treated by adjuvant chemotherapy, and so, to enlarge the panel of prediction of breast cancer GESs.

They have designed a molecular classifier based on a set of 38 genes, which were highly predictive of MR when used together. These 38 genes are listed in Table I below.

Table I

| Gene symbol | Gene title | Genbank # |
| --- | --- | --- |
| BTG2 | BTG family, member 2 | NM_006763 |

(continued)

| Gene symbol | Gene title | Genbank # |
|---|---|---|
| UBE2C | Ubiquitin-conjugating enzyme E2C | BC032677 |
| VEGFA | Vascular endothelial growth factor A | NM_001025366 |
| SLC25A5 | Solute carrier family 25, member 5 | R56229 |
| FABP5 | Fatty acid binding protein 5 | BG282526 |
| ENO1 | Enolase 1, (alpha) | AL833741 |
| PSMB7 | Proteasome (prosome, macropain) subunit, beta type, 7 | R54562 |
| TUBB6 | Tubulin, beta 6 | NM_032525 |
| TACC3 | Transforming, acidic coiled-coil containing protein 3 | BC10607 |
| SURF4 | Surfeit 4 | NM_033161 |
| RHOC | Ras homolog gene family, member C | AK094474 |
| CHCHD2 | Coiled-coil-helix-coiled-coil-helix domain containing 2 | NM_016139 |
| SFRS5 | Splicing factor, arginine/serine-rich 5 | R28509 |
| TNFSF13 | Tumor necrosis factor (ligand) superfamily, member 13 | NM_003808 |
| DDT | D-dopachrome tautomerase | AI936198 |
| C1orf64 | Chromosome 1 open reading frame 64 | AI732325 |
| RAB10 | RAB 10, member RAS oncogene family | AA074077 |
| XIST | X (inactive)-specific transcript | H78857 |
| TUBA4A | Tubulin, alpha 4a | AK054731 |
| PPA1 | Pyrophosphatase (inorganic) 1 | BF694769 |
| C4B | Complement component 4 B | NM_001002029 |
| TUBB2C | Tubulin, beta 2C | BX648521 |
| C3orf37 | Chromosome 3 open reading frame 37 | T65434 |
| GATA3 | GATA binding protein 3 | R16918 |
| PGK1 | Phosphoglycerate kinase 1 | H17787 |
| PPP1CA | Protein phosphatase 1, catalytic subunit, alpha isoform | NM_001008709 |
| BCL2A1 | BCL2-related protein A1 | N28416 |
| IL6ST | Interleukin 6 signal transducer (gp130, oncostatin M receptor) | H04779 |
| PSMB3 | Proteasome (prosome, macropain) subunit, beta type, 3 | R00911 |
| RPN1 | Ribophorin I | NM_002950 |
| ETFB | Electron-transfer-flavoprotein, beta polypeptide | R55473 |
| CYC1 | Cytochrome c-1 | BF569085 |
| POR | P450 (cytochrome) oxidoreductase | CD014011 |
| TUBB3 | Tubulin, beta 3 | T57691 |
| TMED9 | Transmembrane emp24 protein transport containing 9 | NM_017510 |
| TOE1 | Target of EGR1, member 1 (nuclear) | H29584 |
| C17orf37 | Chromosome 17 open reading frame 37 | T84927 |
| PSMA5 | Proteasome (prosome, macropain) subunit, alpha type, 5 | H48425 |

These 38 genes will be designated hereinafter by the gene symbols indicated in Table I, which are those approved by the HUGO Gene Nomenclature Committee (HGNC).

The invention thus relates to a method for evaluating the risk of MR in a breast cancer patient, wherein said method comprises measuring the level of expression of each of the following genes: *BTG2, UBE2C, VEGFA, SLC25A5, FABP5, ENO1, PSMB7, TUBB6, TACC3, SURF4, RHOC, CHCHD2, SFRS5, TNFSF13, DDT, C1orf64, RAB10, XIST TUBA4A, PPA1, C4B, TUBB2C, C3orf37, GATA3, PGK1, PPP1CA, BCL2A1, IL6ST, PSMB3, RPN1, ETFB, CYC1, POR, TUBB3, TMED9, TOE1, C17orf37, PSMA,* in a sample of breast tumoral tissue obtained from said patient.

According to an embodiment of the invention, the level of expression of each of said genes is compared with the mean level of expression of the same gene measured in samples of breast tumoral tissue from a reference group of patients with breast cancer. Preferably, said reference group consists of patients which have unilateral breast cancer, are node-positive, show no evidence of metastasis at diagnosis, are younger than 65 years of age, and have not yet received adjuvant systemic chemotherapy. An example of such a reference group is the training cohort which is described in the examples below.

An overexpression of *UBE2C, VEGFA, SLC25A5, FABP5, ENO1, PSMB7, TUBB6, TACC3, SURF4, RHOC, CHCHD2, DDT, RAB10, TUBA4A, PPA1, TUBB2C, C3orf37, PGK1, PPP1CA, BCL2A1, PSMB3, RPN1, ETFB, CYC1, POR, TUBB3, TMED9, TOE1, C17orf37, PSMA5,* and an underexpression of *BTG2, SFRS5, TNFSF13, C1orf64, XIST, C4B, GATA3, IL6ST,* are correlated with a higher probability of MR.

Methods for measuring the level of expression of a given gene are familiar to one of skill in the art. They typically include methods based on the determination of the level of transcription (i.e. the amount of mRNA produced) of said gene, and methods based on the quantification of the protein encoded by the gene.

Preferably, for carrying out the present invention, the level of expression of the 38 genes listed above is based on the measurement of the level of transcription. This measurement can be performed by various methods which are known in themselves, including in particular quantitative methods involving reverse transcriptase PCR (RT-PCR), such as real-time quantitative RT-PCR (qRT-PCR), and methods involving the use of DNA arrays (macroarrays or microarrays).

Classically, methods involving quantitative RT-PCR comprise a first step wherein cDNA copies of the mRNAs obtained from the biological sample to be tested are produced using reverse transcriptase, and a second step wherein the cDNA copy of the target mRNA is selectively amplified using gene-specific primers. The quantity of PCR amplification product is measured before the PCR reaction reaches its plateau. In these conditions it is proportional to the quantity of the cDNA template (and thus to the quantity of the corresponding mRNA expressed in the sample). In qRT-PCR the quantity of PCR amplification product is monitored in real time during the PCR reaction, allowing an improved quantification (for review on RT-PCR and qRT-PCR, cf. for instance: FREEMAN et al., Biotechniques, 26, 112-22, 24-5, 1999; BUSTIN & MUELLER, Clin Sci (Lond), 109, 365-79, 2005). Parallel PCR amplification of several target sequences can be conducted simultaneously by multiplex PCR.

A "DNA array" is a solid surface (such as a nylon membrane, glass slide, or silicon or ceramic wafer) with an ordered array of spots wherein DNA fragments (which are herein designated as "probes") are attached to it; each spot corresponds to a target gene. DNA arrays can take a variety of forms, differing by the size of the solid surface bearing the spots, the size and the spacing of the spots, and by the nature of the DNA probes. Macroarrays have generally a surface of more than 10 $cm^2$ with spots of 1mm or more; typically they contain at most a few thousand spots.

In cDNA arrays, the probes are generally PCR amplicons obtained from cDNA clones inserts (such as those used to sequence ESTs), or generated from the target gene using gene-specific primers. In oligonucleotide arrays, the probes are oligonucleotides derived from the sequences of their respective targets. Oligonucleotide length may vary from about 20 to about 80 bp. Each gene can be represented by a single oligonucleotide, or by a collection of oligonucleotides, called a probe set.

Regardless of their type, DNA arrays are used essentially in the same manner for measuring the level of transcription of target genes. mRNAs isolated from the biological sample to be tested are converted into their cDNA counterparts, which are labeled (generally with fluorochromes or with radioactivity). The labeled cDNAs are then incubated with the DNA array, in conditions allowing selective hybridization between the cDNA targets and the corresponding probes affixed to the array. After the incubation, non-hybridized cDNAs are removed by washing, and the signal produced by the labeled cDNA targets hybridized at their corresponding probe locations is measured. The intensity of this signal is proportional to the quantity of labeled cDNA hybridized to the probe, and thus to the quantity of the corresponding mRNA expressed in the sample (for review on DNA arrays, cf. for instance: BERTUCCI et al., Hum. Mol. Genet., 8, 1715-22, 1999; CHURCHILL, Nat Genet, 32 Suppl, 490-5, 2002; HELLER, Annu Rev Biomed Eng, 4, 129-53, 2002; RAMASWAMY & GOLUB, J Clin Oncol, 20, 1932-41, 2002; AFFARA, Brief Funct Genomic Proteomic, 2, 7-20, 2003; COPLAND et al., Recent Prog Horm Res, 58, 25-53, 2003)).

The breast tumoral tissue to be analyzed is classically obtained after surgical excision of the tumor followed by a macrodissection step for removing the surrounding healthy tissue. Preferably, it is be rapidly snap-frozen in liquid nitrogen and kept frozen until RNA extraction. Storage reagents, such as RNAlater® (Ambion, Applied Biosystems) can also be used.

Various methods for extracting total RNA from the tumor cells are known in themselves. By way of non-limitative examples, one can cite guanidinium thiocyanate-phenol-chloroform extraction using for instance TRIZOL® reagents (INVITROGEN), or selective binding to silicagel membranes, using for instance the RNeasy® or the AllPrep® kit (QIAGEN).

The integrity of RNA for each sample is assessed for instance with the 2100 Bioanalyzer (Agilent Technologies), using the 'RNA Integrity Number' (RIN) algorithm (SCHROEDER et al., BMC Molecular Biology, 7, 3, 2006) for calculating the RNA integrity. A RIN number higher than 8 is recommended for optimal results, in particular in the case of DNA arrays.

The quality and the concentration of the RNA are assessed by spectrophotometry, using for instance a Nanodrop® spectrophotometer. A 260/280 ratio > 1.8, a 260/230 ratio > 1.9 and a total RNA quantity > 1 $\mu$g are recommended for optimal results.

According to a preferred embodiment of the invention, the level of transcription of the 38 genes listed above is measured, and a risk score (Pi) for a given patient is calculated according to the following equation:

$$Pi = \frac{1}{30}\sum_{i=1}^{30}Gi - \frac{1}{8}\sum_{j=1}^{8}Gj$$

wherein *Gi* represents overexpressed gene standardized expression value and *Gj* represents underexpressed gene standardized expression value.

Overexpressed genes are *UBE2C, VEGFA, SLC25A5, FABP5, ENO1, PSMB7, TUBB6, TACC3, SURF4, RHOC, CHCHD2, DDT, RAB10, TUBA4A, PPA1, TUBB2C, C3orf37, PGK1, PPP1CA, BCL2A1, PSMB3, RPN1, ETFB, CYC1, POR, TUBB3, TMED9, TOE1, C17orf37, PSMA5,* and underexpressed genes are *BTG2, SFRS5, TNFSF13, C1orf64, XIST, C4B, GATA3, IL6ST.*

Standardized expression value is calculated for each gene according to the following equation:

$$G = (E-M)/STD$$

wherein E is the expression value of the patient's gene; M is the mean expression value of the gene in the reference group and STD its standard deviation.

When a DNA array is used, the expression value E for a gene is the log2 transformed intensity of the signal produced by said gene, obtained from the DNA array.

When qRT-PCR is used, the expression value E for a gene is the quantity of PCR amplification product, normalized to a reference gene, [such as *ACTB* (actin beta) or *ACTG1* (actin gamma1)] found by the inventors to have an invariant level of expression (assessed by the coefficient of variation, which was measured prior to the data standardization).

The inventors have calculated the mean and the standard deviation of the expression values of the 38 genes listed above, obtained with a DNA array, from a reference group of 252 patients with breast cancer. They have established the following equation for calculating a risk score for a given patient:

$$Pi = [(POR\text{-}(\text{-}3.73065))/3.83872 + (RPN1\text{-}(\text{-}2.05278))/0.85247 + (RAB10\text{-}(\text{-}5.91109))/3.55600 + (PPA1\text{-}(\text{-}0.44441))/1.01159 + (PGK1\text{-}(\text{-}5.17254))/4.87702 + (C3orf37\text{-}(\text{-}3.19182))/2.34720 + (FABP5\text{-}(\text{-}3.51078))/2.63605 + (RHOC\text{-}(\text{-}1.43741))/0.54387 + (TACC3\text{-}(\text{-}4.13219))/3.54208 + (ENO1\text{-}(0.84342))/0.87915 + (TUBB2C\text{-}(0.42097))/0.81302 + (TUBA4A\text{-}(\text{-}0.68124))/0.92833 + (TOE1\text{-}(\text{-}0.65172))/0.67393 + (UBE2C\text{-}(\text{-}3.02815))/2.83776 + (CYC1\text{-}(\text{-}0.80982))/0.87979 + (TUBB3\text{-}(0.30559))/1.07903 + (C17orf37\text{-}(\text{-}2.16905))/2.84797 + (VEGFA\text{-}(\text{-}6.15262))/4.47508 + (DDT\text{-}(\text{-}1.672061))/1.441825 + (PSMB3\text{-}(\text{-}2.77577))/3.87210 + (ETFB\text{-}(\text{-}0.48413))/1.04725 + (PPP1CA\text{-}(0.00091))/0.94027 + (TMED9\text{-}(\text{-}0.47386))/1.76415 + (CHCHD2\text{-}(0.76363))/0.78549) + (SLC25A5\text{-}(\text{-}1.29147))/2.59668 + (TUBB6\text{-}(\text{-}0.26758))/0.83203 + (PSMA5\text{-}(\text{-}2.68797))/2.57584 + (BCL2A1\text{-}(\text{-}1.25325))/2.30684 + (PSMB7\text{-}(\text{-}1.22944))/0.89287 + (SURF4\text{-}(\text{-}0.78422))/0.88324]/30 - [(XIST\text{-}(\text{-}3.56392))/3.49301 + (GATA3\text{-}(0.55661))/1.81551 + (IL6ST\text{-}(\text{-}3.63925))/4.28647 + (TNFSF13\text{-}(\text{-}3.17162))/2.20540 + (SFRS5\text{-}(\text{-}0.01304))/1.86980 + (C4B\text{-}(1.30273))/1.77614) + (BTG2\text{-}(\text{-}2.95358))/3.57607 + (C1orf64\text{-}(\text{-}6.76347))/6.72459]/8;$$

wherein "GENE SYMBOL" represents the expression value of the corresponding gene in said patient.

It is believed that the estimators (mean and standard deviation) indicated in this equation are broadly usable for calculating the risk score in prospective patients, when the gene-expression values are established using a DNA array.

The 38-gene molecular classifier of the invention allows to evaluate the risk of MR in node-positive breast cancer patients. However, as shown in the examples below, it is also suitable as a prognostic tool for node-negative breast cancer patients. It is more particularly suitable for calculating the risk score in subjects which have not yet begun a chemotherapy.

A risk score lower or equal to -0.65 is indicative of a probability of MR at 5 years of about 97 %; a risk score higher than -0.65 and lower or equal to +0.65 is indicative of a probability of MR at 5 years of about 85 %. A risk score higher than +0.65 is indicative of a probability of MR at 5 years of about 55 %.

The 38-gene molecular classifier of the invention can also be used in conjunction with other prognostic tools for breast cancer patients. In particular, it can be advantageously combined with clinicopathological prognostic indexes such as Nottingham prognostic index (NPI) and Adjuvant! (http://www.adjuvantonline.com).

The present invention also provides kits for using the 38-gene molecular classifier of the invention.

A kit of the invention comprises a combination of reagents allowing to measure the level of expression of each of the 38 genes of said molecular classifier.

Preferably, said kit is designed to measure the level of mRNA of each of these genes. Accordingly, it comprises, for each of the 38 genes, at least one probe or primer that selectively hybridizes with the transcript of said gene, or with the complement thereof.

According to a preferred embodiment of the invention said kit comprises a DNA array. In this case said DNA array will comprise at least 38 different probes, i.e. at least one probe for each of the 38 genes of the molecular classifier.

Said probes can be cDNA probes, or oligonucleotide probes or probe sets.

One of skill in the art can easily find suitable probes, on the basis of the sequence information available for these genes. For instance, other suitable cDNA probes can be found by querying the EST databases with the sequences identified in Table I by their GenBank reference. They can also be obtained by amplification from human cDNA libraries using primers specific of the desired cDNA. Suitable oligonucleotide probes can be easily designed using available software tools (For review, cf. for instance: LI & STORMO, Bioinformatics, 17, 1067-76, 2001; EMRICH et al., Nucleic Acids Res, 31, 3746-50, 2003; ROUILLARD et al., Nucl. Acids Res., 31, 3057-62,2003)

According to another preferred embodiment of the invention, said kit is a PCR kit, which comprises a combination of reagents, allowing specific PCR amplification of the cDNA of each of the 38 genes of the molecular classifier of the invention; these reagents include in particular at least 38 different pairs of primers i.e. at least one specific pair of primers for each of the 38 genes of the molecular classifier.

In the same way as oligonucleotide probes, suitable primers can easily be designed by one of skill in the art, and a broad variety of software tools is available for this purpose (For review, cf. for instance: BINAS, Biotechniques, 29, 988-90, 2000; ROZEN & SKALETSKY, Methods Mol Biol, 132, 365-86, 2000; GORELENKOV et al., Biotechniques, 31, 1326-30, 2001; LEE et al., Appl Bioinformatics, 5, 99-109, 2006; YAMADA et al., Nucleic Acids Res, 34, W665-9, 2006).

Optionally, said primers can be labeled with fluorescent dyes, for use in multiplex PCR assays.

The DNA arrays as well as the PCR kits of the invention may also comprise additional components, for instance, in the case of PCR kits, pairs of primers allowing the specific amplification of the reference gene(s).

The invention will be further illustrated by the following additional description, which exemplifies the use of the molecular classifier of the invention for predicting survival of patients with breast cancer. It should be understood however that this example is given only by way of illustration of the invention and does not constitute in any way a limitation thereof.


## MATERIALS AND METHODS

### Selection and grouping of Patients

### Training cohort (TC)

The training cohort (TC) was composed of 252 women who had unilateral breast cancer, were node-positive, showed no evidence of metastasis at diagnosis, and were younger than 65 years of age. The majority of the studied patients (90.5%) was primarily included in a multicentric phase III clinical trial (PACS01) conducted by investigators from the French Federation of Cancer Centers (FNCLCC) (ROCHÉ et al., J Clin Oncol, 24, 5664-71, 2006). One hundred and twenty eight patients were also part of a previous study; in the present work, follow-up data were actualized (CAMPONE et al., Breast Cancer Res Treat, 109, 491-501, 2007). Twenty four patients (9.5%) followed in the René Gauducheau Cancer Centre (RGCC) were included based on the same inclusion criteria as those used for PACS01 trial.

After surgery, patients received intravenous adjuvant treatment with 5-fluorouracil 500 mg/m$^2$, epirubicin 100 mg/m$^2$ and cyclophosphamide 500 mg/m$^2$ (FEC100) every 21 days for six cycles (PACS01-FEC arm A and RGCC), or three cycles (PACS01-FEC+Docetaxel arm B).

Patients were followed up for metastatic free survival (MFS). No bioclinical heterogeneity between the 3 subpopulations was found. The median follow-up was 7.7 years (range 1.22 - 10.02). During the period, 65 patients showed evidence of distant metastatic relapse (MR) and 47 died.

This substudy of PACS01 trial was reviewed and approved by the ethics committee/institutional (CCPPRB) review board (number 1-97-13). All patients signed informed consent for research purpose.

Clinical and pathological features of the 252 included patients are summarized in Table II below.

Table II

| Variable | All patients (n=252) | PACS01 arm A[a] (n=128) | PACS01 arm B[b] (n=100) | RGCC (n=24) | p |
|---|---|---|---|---|---|
| Age (years) | | | | | |
| ≤35 | 14 | 4 | 9 | 1 | |
| >35 | 238 | 124 | 91 | 23 | 0.143 |
| ER | | | | | |
| - | 58 | 22 | 30 | 6 | |
| + | 181 | 96 | 67 | 18 | |
| md | 13 | 10 | 3 | 0 | 0.091 |
| Scarff-Bloom-Richardson (SBR) grade | | | | | |
| I | 44 | 23 | 17 | 4 | |
| II | 137 | 69 | 59 | 9 | |
| III | 71 | 36 | 24 | 11 | 0.306 |
| Tumor size (mm) | | | | | |
| ≤20 | 94 | 42 | 43 | 9 | |
| >20 | 158 | 86 | 57 | 15 | 0.288 |
| N° positive nodes | | | | | |
| 1-3 | 156 | 78 | 62 | 16 | |
| 4-9 | 79 | 40 | 31 | 8 | |
| >9 | 17 | 10 | 7 | 0 | 0.821 |
| Hormonotherapy | | | | | |
| no | 93 | 52 | 35 | 6 | |
| yes | 159 | 76 | 65 | 18 | 0.311 |
| NPI score | | | | | |
| 1 | 13 | 6 | 7 | 0 | |
| 2 | 136 | 70 | 54 | 12 | |
| 3 | 103 | 52 | 39 | 12 | 0.723 |
| 10 year-OS Adjuvant! score (%) | 55.9 [11.2-89.6] | 55 [11-89] | 58 [14-90] | 54 [32-79] | 0.857 |
| 10 year-OS Adjuvant! score (%) | | | | | |
| ≥80 | 23 | 13 | 10 | 0 | |
| 60-79 | 90 | 43 | 37 | 10 | |
| <60 | 139 | 72 | 53 | 14 | 0.561 |
| 5-year MFS % (95% CI) | 80 (75-85) | 82 (74-88) | 82 (73-88) | 67 (44-82) | 0.150 |
| 5-year OS % (95% CI) | 89 (84-92) | 87 (80-92) | 91 (83-95) | 87 (65-96) | 0.680 |
| a: FEC; b: FEC-Docetaxel; Continuous variables: median [range]; md: missing data | | | | | |

## Validation cohort (VC)

External validation was based on independent external genomic data sets. The selection of these breast cancer cohorts was based on the following inclusion criteria: available genomic profiles, node-positive and MR well defined in order to use the same outcome definition as the one used in the training set. Hence, we extracted three node-positive subcohorts (VC1 to VC3) from three published cohorts (VAN DE VIJVER et al., N Engl J Med, 347, 1999-2009,2002; SOTIRIOU et al., Proc Natl Acad Sci USA, 100, 10393-98, 2003; SOTIRIOU et al., J Natl Cancer Inst, 98,262-72, 2006).

These gene-expression data sets were generated from three different microarray platforms (Agilent®, Affymetrix®, cDNA custom array). In Van de Vijver's subcohort (VC1), 62.5% of patients had received adjuvant systemic chemotherapy. Sixty percents of Sotiriou's subcohort published in 2003 (VC2) had received a systemic treatment consisting in 6 cycles of cyclophosphamide, methotrexate, and 5-fluorouracil (CMF). All patients included in Sotiriou study published in 2006 (VC3) had received hormonal therapy alone (Tamoxifen) [SOTIRIOU 2006, cited above]. No statistical heterogeneity was found between TC and VC1+VC2+VC3 for available data (ER (estrogen receptor) and 5- year MFS).

**Exploratory cohorts (EC)**

To explore performance capacities of 38-GES, a complementary study was conducted on breast cancer patients who did not fulfill 38-GES establishment inclusion criteria. Three subcohorts (EC1 to EC3) and three cohorts of breast cancer patients (EC4 to EC6) were used (VAN DE VIJVER et al. , 2002; SOTIRIOU et al., 2003; SOTIRIOU et al., 2006, cited above; WANG et al., The Lancet, 365, 671-79, 2005; PAWITAN et al., Breast Cancer Res; 7: R953-R964, 2005; IVSHINA et al. Cancer Res, 66: 10292-301, 2006). We used MR as endpoint in the four node-negative groups (EC1 to EC4) and unspecified event (UE) (local relapse, MR, cancer death) in the two node-mixed cohorts (EC5 and EC6). No heterogeneity was found for available data between TC and EC1+EC2+EC3+EC4 or between TC and EC5+EC6.
The methodological distribution and status of the studied cohorts are summarized in Table III below

Table III

| code | Data sets | Nodal status | | | Clinical outcome | |
|---|---|---|---|---|---|---|
| | | N+ | N- | mixed | MR | UE |
| | **TRAINING COHORT** | | | | | |
| TC | our study | 252 | | | 65 | |
| | *Total* | **252** | | | **65** | |
| | **VALIDATION COHORT** | | | | | |
| VC1 | Van de Vijver et al | 144 | | | 47 | |
| VC2 | Sotiriou et al, 2003 | 53 | | | 23 | |
| VC3 | Sotiriou et al, 2006 | 27 | | | 7 | |
| | *Total* | **224** | | | **77** | |
| | **EXPLORATORY COHORTS** | | | | | |
| EC1 | Van de Vijver et al | | **151** | | 54 | |
| EC2 | Sotiriou et al, 2003 | | 46 | | 7 | |
| EC3 | Sotiriou et al, 2006 | | 149 | | 33 | |
| EC4 | Wang et al | | 286 | | 107 | |
| | *Subtotal* | | **632** | | **201** | |
| EC5 | Pawitan et al | | | 159 | | 40 |
| EC6 | Ivshina et al | | | 249 | | 89 |
| | *Subtotal* | | | **408** | | **129** |
| MR: metastatic relapse; UE: unspecified event: local relapse, MR or cancer death | | | | | | |

**Tumor tissue samples and RNA extraction**

Tumor tissue samples were surgically collected, macrodissected and immediately snapfrozen in liquid nitrogen and stored until RNA extraction.
Total RNA was prepared by the CsCl-cushion, as described by CHIRGWIN et al. (Biochemistry; 18, 5294- 99, 1979) from the tumor tissue samples obtained after surgery. The quality and the quantity of RNA samples were evaluated by Agilent 2100 Bioanalyser RNA LabChip kit (Agilent Technologies, Palo Alto, CA, USA).

**Nylon microarray technology**

In the present study, we used nylon microarrays manufactured in our 1ab, which contained 8,032 unique sequenced-verified cDNA clones, representing 5,776 distinct genes, chosen using the expressed sequence tag (EST) database from the NCBI. The same microarray tools were used and described in previous works (MAGRANGEAS et al., Blood, 101, 4998-5006, 2003; CAMPONE et al., 2007, cited above; DECAUX et al., J Clin Oncol, 26,2008).

Five $\mu$g of RNA were labeled by reverse transcription and incorporation of [ -33P]dCTP. Then nylon microarrays were hybridized with the labeled cDNAs probes as previously described (MAGRANGEAS et al., 2003, cited above).

DNA microarrays were scanned at 25-$\mu$m resolution using a Fuji BAS 5000 image plate system (Raytest, Paris, France). The hybridization signals were quantified using ArrayGauge software v.1.3 (Fuji Ltd, Tokyo, Japan). For each membrane, the raw data were background noise subtracted (median value of negative controls $\pm$ 6 standard deviation (std)), then normalized by global intensity of hybridization. Patient data were adjusted by the amount of PCR product spotted onto the membrane, $\log_2$-transformed and finally standardized (mean=0; std=1). Only genes well measured for 70% of patients were retained.

**Establishment of the predictive gene expression signature**

**Data analysis**

Genes selection

Genes were selected by means of resampling and univariate Cox permutations techniques in order to minimize overfitting and to maximize stability of gene list for MFS prediction.

We created 100 different random samples composed of the 2/3 of TC. For each of these 100 subsamples i (i=1 to 100), we determined an unbiased predictor (pi) with internal robust cross-validation (leave-one-out cross-validation method). One-hundred pi (i=1 to 100) were thus determined. In order to increase stability of final gene list (n), only genes present in at least 50% of the unbiased resampled gene predictors pi were retained to build the n-GES.

GES determination

The 38-GES score was defined as the difference between the average expression of the up-regulated genes and the average expression of the down-regulated genes in the TC. No weight was assigned to genes in order to limit overfitting. Standardization of gene distributions permitted extrapolation of fully determined 38-GES (including cutoff) onto other cohorts. Optimal cutoff between 38-GES low-risk and high-risk groups was determined as the value with minimal Akaike Information Criterion (AIC).

GES validation

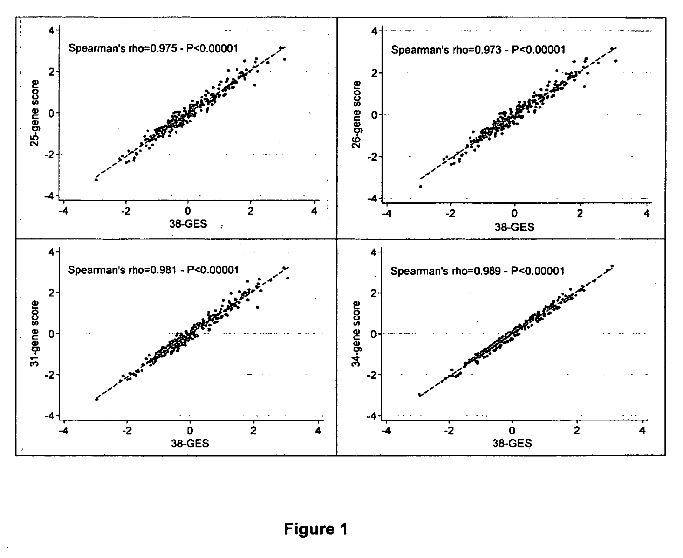

As the 38-GES genes were not always present in the different validation cohorts (VCs) and exploratory cohorts (ECs) (n = 25 to 38), it was necessary to evaluate if the different 38-GES subscores were significantly correlated with 38-GES complete score in the training cohort (TC). All the n-GES subscores (n = 25 [pooled VCs: 1 to 3; pooled ECs: 1 to 4], 26 [VC2 and EC2], 31 [VC1 and EC1], 34 [VC3, EC3 and EC4]) were highly correlated with 38-GES score (Spearman's rho $\geq$ 0.973), as shown in Figure 1. Subscores were then used for risk group determination in each individual test cohort. They were calculated as indicated in the following panel.

---

*Panel:* Calculation of 38-GES subscore

$$\text{n-GES} = \frac{1}{p}\sum_{i=1}^{p} Gi - \frac{1}{q}\sum_{j=1}^{q} Gj \qquad \text{with n = 25, 26, 31, 34}$$

*Gi* and *Gj* represent overexpressed and underexpressed gene-expression standardized values, respectively. No weight was assigned to genes in order to limit overfitting.

For n = 25, 26, 31 and 34, p was 19, 20, 25, 27 and q was 6, 6, 6, 7, respectively.

For n = 25, 26, 31 and 34, n-GES optimal cutoff in TC: was -0.264, - 0.245, 0.065, 0.065, respectively.

---

Each subscore optimal cutoff was defined as the value with minimal AIC in training cohort.

### Survival analysis and endpoints

Time from surgery to MR (primary endpoint) was retained for GES establishment. Overall survival (OS), defined as time from surgery to death from any cause, was used to reinforce MFS analysis in TC. Survival curves were plotted according to Kaplan-Meier method and compared by means of log-rank test (or Wilcoxon log-rank test if number of events was small, in order to give greater weight to earlier events). For each univariate survival analysis, 10,000 permutation survival analyses were performed to ensure p-values were not optimized by overfitting. Only parameters with permutation $p<0.05$ were entered into the forward Cox proportional hazards model. Proportional hazards assumption was verified for the final models by means of Schoenfeld residuals study and -ln(-ln(survival)) curves. Because time to distant metastasis was not specified for EC5 and EC6, time to the first cancer event (local relapse, MR or death) was used.

### Sensitivity, specificity

Receiver operating characteristic (ROC) analysis with MR within 5 years as a defining point was computed. Patients who did not have 5-year follow-up were excluded from ROC analysis. The area under the curve (AUC) was used as a measure of the signature performance in the test set. Time-dependent ROC curves were calculated for 38-GES , Nottingham prognostic index (NPI) and Adjuvant! (HEAGERTY et al., Biometrics, 56, 337-44,2000).

### Clinical Risk Classification

The aim of this new validation study was to examine whether the 38-GES had prognostic value independent of the best clinical risk classifications (NPI and Adjuvant!) and could improve classifcation of subset of patients which remains at intermediate risk with this two clinical scores. Final study was thus focused onto these particular patients. In the first part of the study, it was decided to use for each of the compared parameters its optimal cut-off in order to not advantage 38-GES in multivariate analyses. Moreover, to avoid 38-GES adjusted hazard ratio (HR) to be significant by chance with an arbitrary cutoff, 38-GES HR was calculated adjusted on NPI and Adjuvant! over all their range. Once 38-GES prognostic value proved, in the second part of the study, it was decided to use pragmatic cutoffs.

### Nottingham prognostic values and classification

NPI scores were calculated as follows: [0.2 x tumor size (cm)] + SBR grade (1, 2, or 3) + lymph node stage (1, 2, or 3). Tumor size was defined as the longest diameter of the tumor. Lymph node stage was 1 if lymph node negative, 2 if 1 to 3 nodes involved, and 3 if >3 nodes involved. NPI scores : <3.4 (good prognostic group: 80% 15-yr OS); scores of 3.4 to 5.4 (moderate prognostic group: 42% 15-year OS); scores >5.4 (poor prognostic group: 13% 15-year OS).

### Adjuvant! Online prognostic values and classification

The Adjuvant! software (http://www.adjuvantonline.com) calculates 10-year survival probability based on the patient's age, tumor size and grade, ER and nodal status. Although it is possible to use the outcomes produced by Adjuvant! software as a continuous variable, clinical decision making assumes a dichotomization into a low- and a high-risk group.

### Clinicogenomic model establishment and classification

Firstly, multivariate Cox regression analysis was used to determine if n-GES added independent prognostic information to NPI and Adjuvant!. To rule out the possibility that our results were dependent on the choice of clinical cutoff, we carried out a sensitivity analysis in which we varied the NPI raw score that defined low-risk, as predicted by NPI, from 3.5 (in which case most patients were classified in the high-risk group) to 7 (in which case most patients were classified in the low-risk group). For Adjuvant!, the same analysis as described above was done from 20% (in which case most patients were classified in the low-clinical risk group) to 80% (in which case most patients were classified in the high-clinical risk group). Secondly, we focused on patients with NPI and Adjuvant! intermediate-risk: score 2 and 10-year OS probabilities between 50% and 80%, respectively. For those patients, the therapeutic decision could be facilitated by an appropriate refining, for example to orientate 38-GES high-risk patients toward new clinical trials.

### Statistical **analysis**

All analyses were performed with SAS 9.1 (SAS Institute Ins., Cary, NC), Stata 10.0 SE (Stata Corp., College Station, TX), R software (version 2.5.1) and BRB ArrayTools developed by Dr. R. Simon and A. Peng (Bethesda, MD;

2003) (http://linus.nci.nih.gov/BRB-ArrayTools.html).

Statistical significance of each of the n-GES genes was assessed in each of the studied cohorts and subcohorts by univariate Cox model.

## RESULTS

## EXAMPLE 1: CONSTRUCTION OF A 38-GENE CLASSIFIER

### Establishment of the predictive gene expression signature

Thirty eight genes were kept. These genes are listed in Table IV below. Control of false discovery rate (FDR) among the differentially expressed genes (DUPUY and SIMON, J Natl Cancer Inst, 99, 147-157, 2007) was assessed in BRB-ArrayTools by 10,000 permutations: p were <0.008 and FDR was <18% for all genes but one (p=0.014, FDR=24%).

Table IV

| % model n=100 | P-permutation n=10,000 | Gene symbol | MR sense | Genbank # |
|---|---|---|---|---|
| 99 | <1.10-7 | BTG2 | - | NM_006763 |
| 96 | <1.10-7 | UBE2C | + | BC032677 |
| 96 | 0.0001 | VEGFA | + | NM_001025366 |
| 94 | 0.0041 | SLC25A5 | + | R56229 |
| 93 | 0.0010 | FABP5 | + | BG282526 |
| 89 | 0.0004 | ENO1 | + | AL833741 |
| 86 | 0.0004 | PSMB7 | + | R54562 |
| 81 | 0.0003 | TUBB6 | + | NM_032525 |
| 81 | 0.0016 | TACC3 | + | BC106071 |
| 80 | 0.0001 | SURF4 | + | NM_033161 |
| 77 | 0.0007 | RHOC | + | AK094474 |
| 76 | 0.0004 | CHCHD2 | + | NM_016139 |
| 71 | 0.0011 | SFRS5 | - | R28509 |
| 70 | 0.0004 | TNFSF13 | - | NM_003808 |
| 70 | 0.0024 | DDT | + | AI936198 |
| 67 | 0.0003 | C1orf64 | - | AI732325 |
| 67 | 0.0040 | RAB10 | + | AA074077 |
| 66 | 0.0011 | XIST | - | H78857 |
| 65 | 0.0017 | TUBA4A | + | AK054731 |
| 63 | 0.0041 | PPA1 | + | BF694769 |
| 62 | 0.0011 | C4B | - | NM_001002029 |
| 62 | 0.0018 | TUBB2C | + | BX648521 |
| 62 | 0.0071 | C3orf37 | + | T65434 |
| 60 | 0.0017 | GATA3 | - | R16918 |
| 60 | 0.0039 | PGK1 | + | H17787 |
| 59 | 0.0014 | PPP1CA | + | NM_001008709 |
| 59 | 0.0022 | BCL2A1 | + | N28416 |
| 58 | 0.0005 | IL6ST | - | H04779 |
| 58 | 0.0017 | PSMB3 | + | R00911 |
| 58 | 0.0020 | RPN1 | + | NM_002950 |
| 57 | 0.0033 | ETFB | + | R55473 |
| 56 | 0.0006 | CYC1 | + | BF569085 |
| 56 | 0.0144 | POR | + | CD014011 |
| 55 | 0.0016 | TUBB3 | + | T57691 |
| 53 | 0.0060 | TMED9 | + | NM_017510 |
| 52 | 0.0036 | TOE1 | + | H29584 |
| 52 | 0.0045 | C17orf37 | + | T84927 |
| 51 | 0.0073 | PSMA5 | + | H48425 |

As shown in Table IV, 30 upregulated genes (UBE2C, VEGFA, SLC25A5, FABP5, ENO1, PSMB7, TUBB6, TACC3, SURF4, RHOC, CHCHD2, DDT, RAB10, TUBA4A, PPA1, TUBB2C, C3orf37, PGK1, PPP1CA, BCL2A1,

*PSMB3, RPN1, ETFB, CYC1, POR, TUBB3, TMED9, TOE1, C17orf37, PSMA5*) and 8 down-regulated genes (*BTG2, SFRS5, TNFSF13, C1orf64, XIST, C4B, GATA3, IL6ST*) were identified.

## Molecular biology of the markers

Several biological processes were identified by using Gene Ontology database (http://www.geneontology.org/). These processes are listed in Table V below.

Table V

| Gene symbol | Biological process (Gene Ontology) |
|---|---|
| BTG2 | DNA repair, transcription, regulation of transcription DNA-dependent, negative regulation of cell proliferation |
| UBE2C | Ubiquitin proteasome system, cell cycle |
| VEGFA | Angiogenesis, regulation of progression, signal transduction |
| SLC25A5 | Transport, mitochondrial transport |
| FABP5 | Lipid metabolic process, transport |
| ENO1 | Glycolysis, transcription |
| PSMB7 | Ubiquitin proteasome system, cell cycle |
| TUBB6 | Microtubule-based process, microtubule-based movement, protein polymerization |
| TACC3 | Progression, response to stress, hemopoiesis |
| SURF4 | Unknown |
| RHOC | Glycolysis, signal transduction, regulation NF-kappaB cascade |
| CHCHD2 | Unknown |
| SIRS5 | mRNA splice site selection, mRNA processing, RNA splicing |
| TNFSF13 | Angiogenesis, cell proliferation, apoptosis, immune response |
| DDT | Melanin biosynthetic process from tyrosin |
| C1orf64 | Unknown |
| RAB10 | Regulation of transcription DNA-dependent, intracellular protein transport, nucleocytoplasmic transport, signal transduction, small GTPase mediated signal transduction |
| XIST | Unknown |
| TUBA4A | Microtubule-based process, microtubule-based movement, protein polymerization |
| PPA1 | Phosphate metabolic process |
| C4B | Inflammatory response, complement adivation, innate immune response |
| TUBB2C | Cell motility, microtubule-based process, microtubule-based movement, natural killer cell mediated cytotoxicity, protein polymerization |
| C3orf37 | Unknown |
| GA TA3 | Cell fate determination, transcription, regulation of transcription DNA-dependent, transcription from RNA polymerase II promoter, defense response |
| PGK1 | Glycolysis, phosphorylation |
| PPP1CA | Carbohydrate metabolic process, protein amino-acid dephosphorylation, cell cycle |
| BCL2A1 | Anti-apoptosis, regulation of apoptosis |
| IL6ST | Glycogen metabolic process, immune response, signal transduction, cell surface receptor linked signal transduction, positive regulation of cell proliferation |
| PSMB3 | Ubiquitin-dependent protein catabolic process |

(continued)

| Gene symbol | Biological process (Gene Ontology) |
|---|---|
| RPN1 | Protein amino-acid glycosylation |
| ETFB | Electron transport, transport |
| CYC1 | Electron transport, transport |
| POR | Electron transport |
| TUBB3 | Microtubule-based process, microtubule-based movement, mitosis, signal transduction, G-protein coupled receptor protein signaling pathway |
| TMED9 | Transport |
| TOE1 | Negative regulation of transcription from RNA polymerase II promoter |
| C17orf37 | Cell redox homeostasis, protein homooligomerization |
| PSMA5 | Ubiquitin-dependent protein catabolic process |

In order of representation, these were: transcription (6 genes), transport (6), ubiquitin-proteasome system (4), microtubule-based process (4), signal transduction (4), proliferation (3), cell cycle (3), glycolysis (3), immune response (3), electron transport (3), apoptosis (2), angiogenesis (2) and progression (2). Literature mining showed that the majority of these genes are biologically relevant since they represent functional categories that are reported to influence breast cancer evolution and sensitivity to chemotherapy.

**EXAMPLE 2: COMPARISON OF NOTTINGHAM PROGNOSTIC INDEX, ADJUVANT! ONLINE AND 38-GES CLASSIFICATION**

The 38-GES relapse score is calculated as defined below:

$$38\text{-GES} = \frac{1}{30}\sum_{i=1}^{30} Gi - \frac{1}{8}\sum_{j=1}^{8} Gj$$

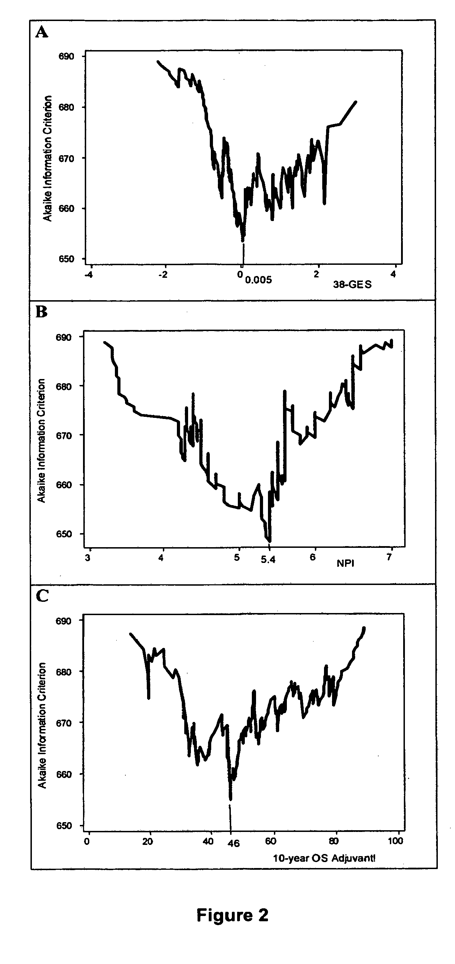

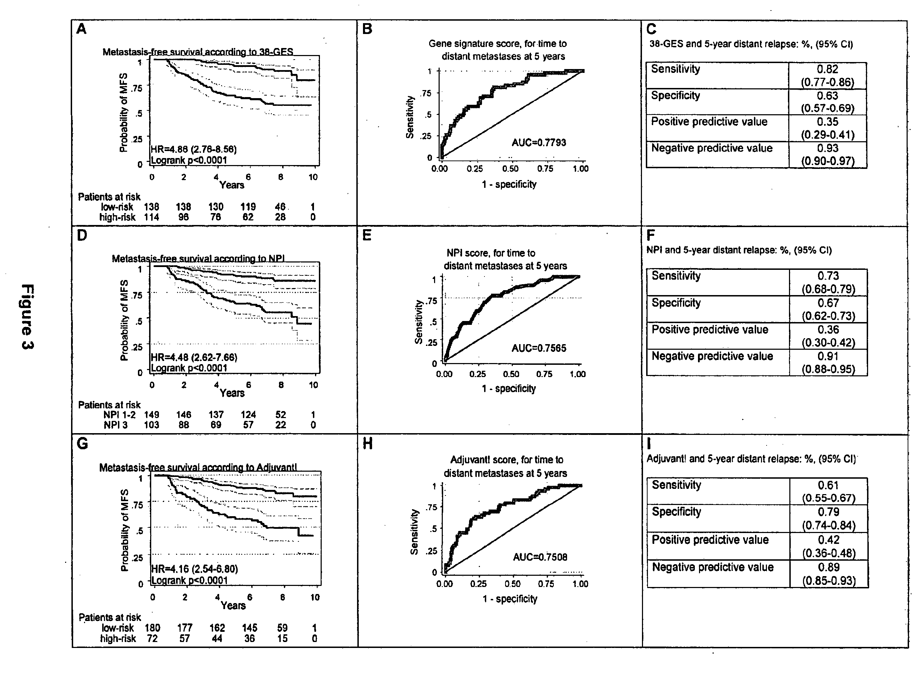

*Gi* and *Gj* represent overexpressed and underexpressed gene-expression standardized values, respectively.
Optimal cutoff determination for the raw value of 38-GES (A), NPI (B) and Adjuvant! (C) scores were defined as the value with minimal AIC in training cohort). The results are shown in Figure 2. For 38-GES, optimal cutoff was 0.005. For NPI, 5.4 was found which is the "official" value defining high-risk group; this was not totally unexpected in a node-positive population. For Adjuvant!, 46% 10-year overall survival was found.
The MFS, ROC curves and characteristics for 38-GES (A, B, C), Nottingham prognostic index (D, E, F) and Adjuvant! (G, H, I) scores in training cohort are shown in Figure 3. Optimal MFS univariate HR (high-risk vs low-risk) for 38- GES, NPI and Adjuvant! were 4.86 (95% CI: 2.76-8.56; p<0.0001), 4.48 (95% CI: 2.62-7.66; p<0.0001), 4.16 (95% CI: 2.54-6.80; p<0.0001), respectively. ROC analysis for MR within 5 years, as a defining point, showed that 38-GES global predictive performance (AUC=0.779) was higher than NPI's one (AUC=0.757) and Adjuvant!'s one (AUC=0.751) with higher sensitivity. Time-dependent ROC curves confirmed that the global predictive performance of the 38-GES was better than that of NPI and Adjuvant!. AIC of the 3 classification schemes showed that the classification power of 38-GES (AIC=641.44) was better than the NPI's one (AIC=649.58), which itself was better than Adjuvant!'s one (AIC=653.79) to explain the MFS.
The Univariate (original data set and permutation test: n=10,000) and multivariate (original data set) MFS analysis for 38-GES, NPI and Adjuvant! are shown in Table VI. MFS multivariate Cox regression analysis proved that 38-GES added very significant and independent prognostic information to NPI (adjusted HR=3.30, p<0.0001) and Adjuvant! (adjusted HR=3.40, p<0.0001).

Table VI

| Variable | HR | 95% CI HR | Univariate p | Univariate permutation p N=10,000 | Adjusted HR | 95% CI adjusted HR | Multivariate p |
|---|---|---|---|---|---|---|---|
| Age (years) ≤35 vs > 35 | 1.35 | 0.54-3.35 | 0.5222 | 0.4601 | | | |
| Tumor size (> 20mm vs ≤20mm) | 1.87 | 1.07-3.25 | 0.0271 | 0.0143 | | | |
| N° positive nodes | | | | | | | |
| 4-9 vs 1-3 | 2.58 | 1.53-4.35 | 0.0004 | | | | |
| >9 vs 1-3 | 3.86 | 1.74-8.54 | 0.0009 | 0.0001 | | | |
| SBR grade | | | | | | | |
| II vs I | 5.93 | 1.42-24.70 | 0.0147 | 0.0001 | | | |
| III vs I | 12.63 | 3.02-52.88 | 0.0005 | | | | |
| ER (+ vs -) | 0.39 | 0.24-0.66 | 0.0004 | 0.0007 | | | |
| Docetaxel (yes vs no) | 0.85 | 0.51-1.41 | 0.5353 | 0.5327 | | | |
| Hormonotherapy (yes vs no) | 0.47 | 0.29-0.77 | 0.0025 | 0.0004 | | | |
| NPI score (3 vs 1+2) | 4.48 | 2.62-7.66 | <0.0001 | <0.0001 | 2.95 | 1.68-5.21 | <0.0001 |
| NPI 1, 2, 3 | 4.30 | 2.56-7.23 | <0.0001 | <0.0001 | | | |
| 10-year OS Adjuvant! score | 0.96 | 0.94-0.97 | <0.0001 | <0.0001 | | | |
| 10-year OS Adjuvant! score (<46% vs ≥46%) | 4.16 | 2.55-6.80 | <0.0001 | <0.0001 | 2.61 | 1.54-4.41 | <0.0001 |
| 10-year OS Adjuvant! score (< 60% vs 60-79% vs ≥ 80%) | 2.67 | 1.62-4.42 | 0.0001 | <0.0001 | | | |
| 38-GES | 2.45 | 1.90-3.16 | <0.0001 | <0.0001 | | | |
| 38-GES (high-risk vs low-risk) | 4.86 | 2.76-8.56 | <0.0001 | <0.0001 | 3.30[a] | 1.81-5.99 | <0.0001 |
| | | | | | 3.40[b] | 1.85-6.24 | <0.0001 |

HR: hazard ratio; a: model with NPI; b: model with Adjuvant!

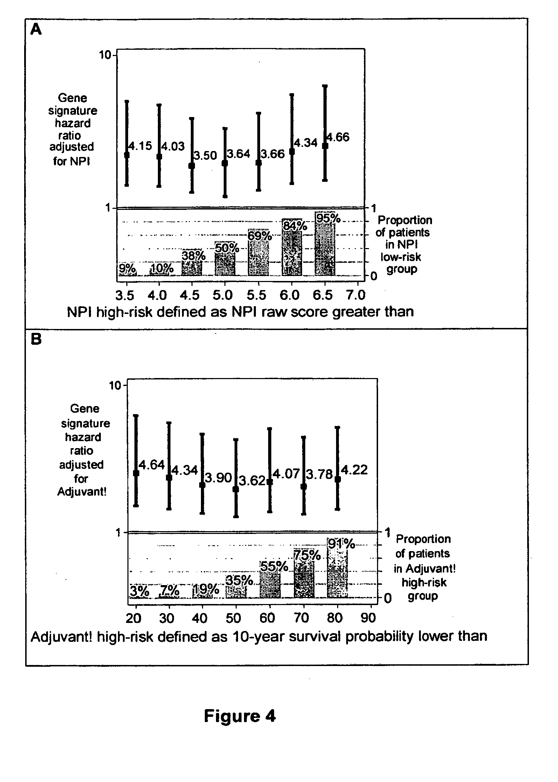

Figure 4 shows the hazard ratios (and 95% CI) for 38-GES high-risk vs low-risk, adjusted for clinical risk groups based on NPI raw score with cutoff varying from 3.5 to 7 (A) and 10-year overall survival Adjuvant! score with cutoff varying from 20% to 80% (B). X-axis indicates the cutoff points which define high clinical risk for adjustment. 38-GES hazard ratio adjusted for NPI (A) and Adjuvant! (B) remained highly significant whatever the cutoff chosen. Squares, HR; vertical lines, 95%CI; shaded bars, proportion of patients in NPI low-risk or Adjuvant! high-risk group as defined by each cutoff. These results show that the 38-GES kept its independent prognostic value whatever the definition of NPI and Adjuvant! high-risk group was.

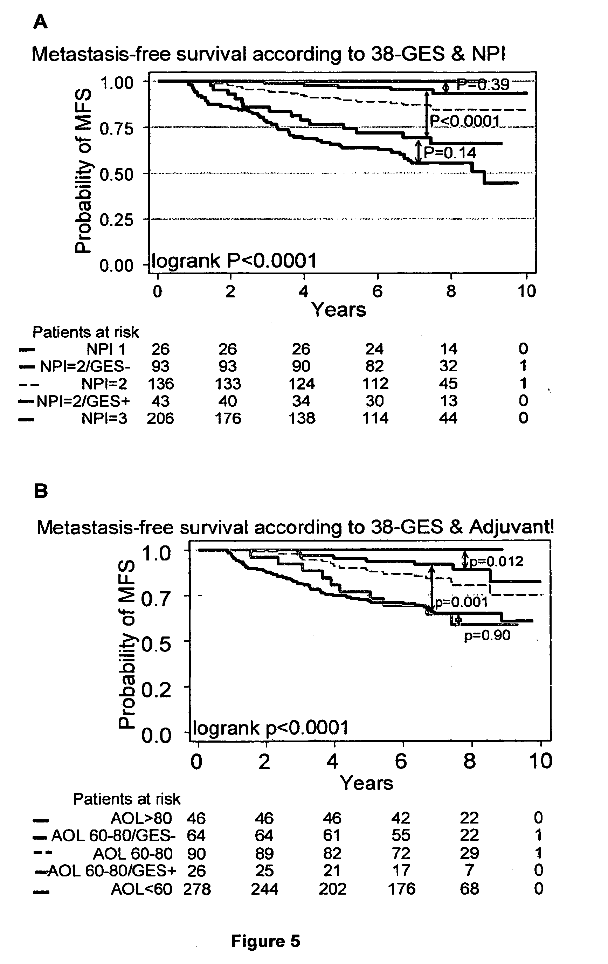

Figure 5 shows the MFS according to 38-GES vs NPI (A) and Adjuvant! (B) classifications. These results show that 38-GES substratifies NPI and Adjuvant! intermediate-risk patients.

The same successful stratification was obtained for NPI and Adjuvant! high-risk patients, as shown in Table VII below.

Table VII

|  | N | 5-year MFS % (95% CI) | Logrank p |
|---|---|---|---|
| NPI=1 |  | 100 (-) |  |
| 38-GES low-risk | 13 |  |  |
| 38-GES high-risk | 0 |  |  |
| NPI=2 |  | 90.4 (84.1-94.3) |  |
| 38-GES low-risk | 93 | 96.7 (90.2-98.9) |  |
| 38-GES hiqh-risk | 43 | 76.7 (61.1-86.8) | <0.0001 |
| NPI=3 |  | 64.9 (54.8-73.2) |  |
| 38-GES low-risk | 32 | 80.9 (62.4-90.9) |  |
| 38-GES high-risk | 71 | 57.7 (45.3-68.2) | 0.021 |
| 10-year OS Adjuvant!≥80% |  | 100 (-) |  |
| 38-GES low-risk | 21 |  |  |
| 38-GES high-risk | 2 |  |  |
| 10-year OS Adjuvant! 60-79% |  | 88.9 (80.3-93.9) |  |
| 38-GES low-risk | 64 | 93.7 (84.1-97.6) |  |
| 38-GES high-risk | 26 | 76.9 (55.7-88.9) | 0.003 |
| 10-year OS Adjuvant! <60% |  | 71.8 (63.5-78.5) |  |
| 38-GES low-risk | 53 | 90.3 (78.3-95.9) |  |
| 38-GES high-risk | 86 | 60.4 (49.3-69.8) | 0.001 |

NPI 2 patients with 5- year MFS=90.4% could be separated in 2/3 with 5-year MFS=96.7% close to NPI 1 and 1/3 5-year MFS=76.7% close to NPI 3 (HR=6.97, p<0.0001). Similarly, Adjuvant! 60-79% 10-year OS patients with 5-year MFS=88.9% could be separated in 2/3 with 5-year MFS=93.7% close to Adjuvant! [3]80% 10-year OS group and 1/3 5-year MFS=76.9% close to Adjuvant! <60% 10-year OS group (HR=4.34, p<0.0001).

## EXAMPLE 3: EXTERNAL VALIDATION OF THE 38-GES

As the 38 genes were not always present in the different test cohorts (n=25 to 38), it was necessary to make sure that the different 38-GES subscores were correlated enough and significantly with 38-GES complete score in the training cohort. All the n-GES subscores (n=25, 26, 31, 34) were highly correlated with 38-GES score, with Rho coefficients ranging from 0.973 to 0.989 with all p-values<0.00001. Subscores were then used for risk group determination in each individual test cohort. Each sub-score optimal cutoff was defined as the value with minimal AIC in training cohort.

They were calculated as follows:

$$\text{n-GES} = \frac{1}{p}\sum_{i=1}^{p} Gi - \frac{1}{q}\sum_{j=1}^{q} Gj$$

with n=25, 26, 31, or 34

G$i$ and G$j$ represent overexpressed and underexpressed gene-expression standardized values, respectively. No weight was assigned to genes in order to limit overfitting. For n=25, 26, 31 and 34, $p$ was 19, 20, 25, 27 and q was 6, 6, 6, 7 respectively.

For n=25, 26, 31 and 34, n-GES optimal cutoff in TC was -0.264, -0.245, 0.065,0.065, respectively.

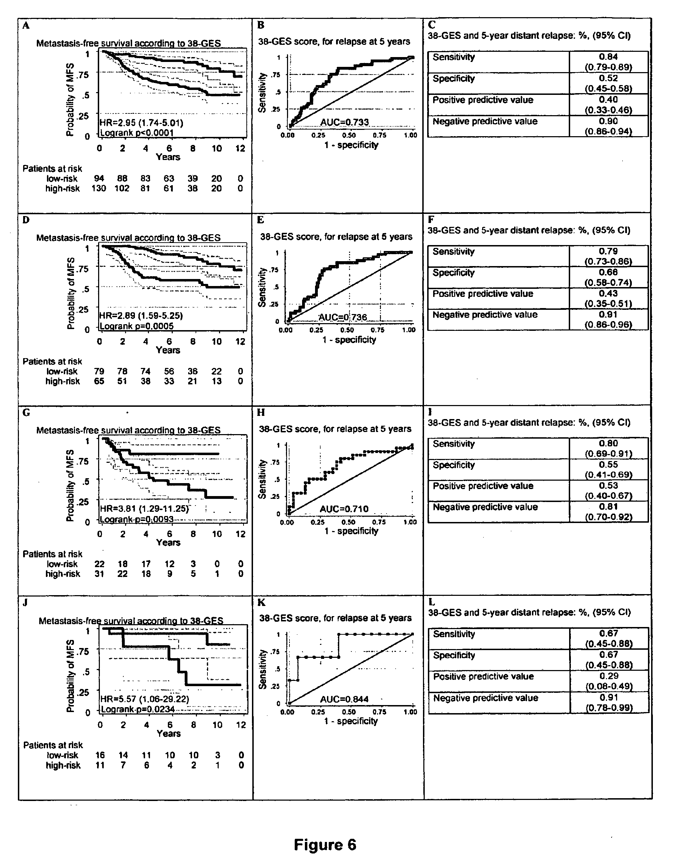

Figure 6 represents the MFS, ROC curves and characteristics in pooled (A,B,C) and separate node-positive VCs: VC1 (D,E,F), VC2 (G,H,I) and VC3 (J,K,L). These results show that 38-GES significantly predicted MFS in pooled node-positive VC (HR=2.95, 95% CI: 1.74-5.01, p<0.0001) with good sensitivity (0.84) and good global predictive performance (AUC=0.73).

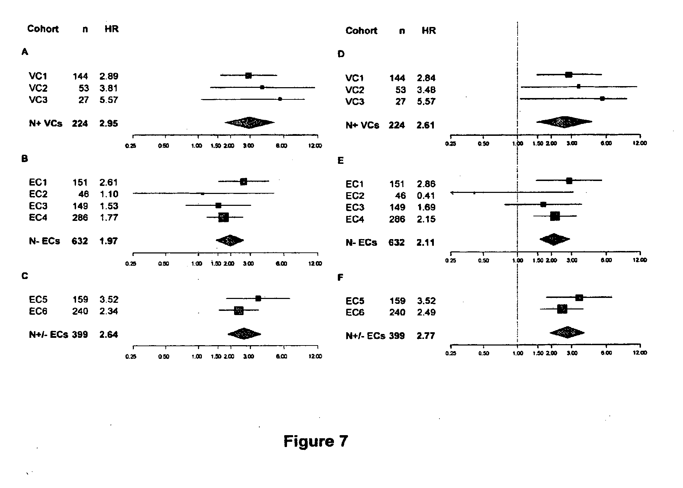

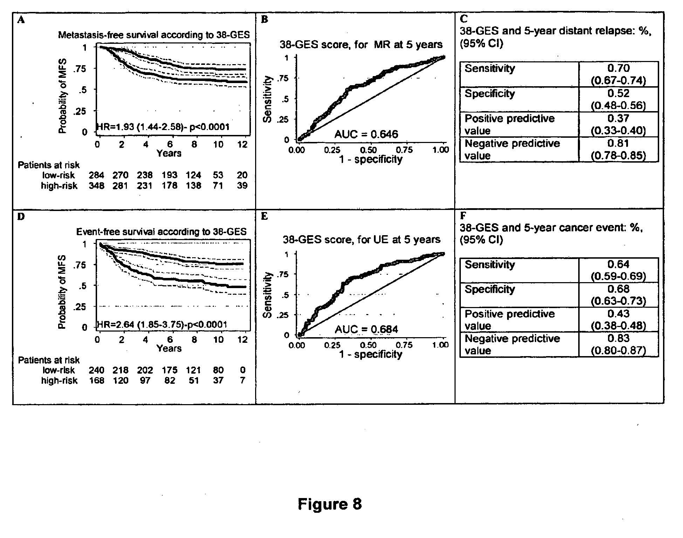

Figure 7 represents the Forest-plot diagram of 38-GES MFS hazard ratios (HR) and 95% confidence intervals in high-risk vs low-risk groups for VCs and ECs: unadjusted HR (A,B,C) and ER adjusted HR (D,E,F). In F, as ER status was unknown in EC5, global EC5+EC6 adjusted HR (2.77) was estimated as the mean of extreme hypothesis for ER status in EC5 (all patients ER- / all patients).Even when ER adjusted, 38-GES kept strong predictive value in pooled VC (HR=2.63, 95% CI: 1.50-4.62, p<0.0001) and separate VCs. Figure 8 represents MFS, ROC curves and characteristics in pooled node-negative ECs (EC1 to EC4) (A,B,C) and pooled node mixed ECs (EC5, EC6) (D,E,F). These results show that 38-GES significantly predicted time to distant metastasis in pooled node-negative exploratory cohort (ER adjusted HR=2.11, 95% CI: 1.53-2.92, p<0.0001) and time to disease event (local relapse, metastasis and death) in mixed node cohorts (ER adjusted HR=2.77, 95% CI: 1.93-3.97, p<0.0001).

Table VIII below shows the classification of the 38-GES genes based on number of significant Cox univariate analysis p values in the different studied breast cancer cohorts.

## Table VIII

| n° of cohorts with significant expression | gene | TC | VC1 | VC2 | VC3 | EC1 | EC2 | EC3 | EC4 | EC5 | EC6 |
|---|---|---|---|---|---|---|---|---|---|---|---|
| 7 | UBE2C | 0.00005 | 0.00113 | ns | ns1 | 0.00024 | ns | 0.01962 | 0.00066 | 0.00051 | 0.00025 |
| 6 | TACC3 | 0.00127 | 0.02032 | ns | ns | 0.00033 | ns | ns2 | 0.03920 | 0.00100 | 0.00018 |
| 5 | IL6ST | 0.00034 | 0.01533 | ns | ns2 | 0.03427 | ns | ns | 0.00812 | 0.00076 | ns |
| 5 | TUBB2C | 0.00096 | ns1 | md | ns | 0.00929 | md | 0.04814 | ns | 0.00003 | 0.00101 |
| 5 | TUBB3 | 0.00146 | ns | md | ns | ns | md | 0.04956 | 0.03492 | < 0.00001 | 0.00083 |
| 5 | PGK1 | 0.00322 | 0.00048 | md | ns | 0.00006 | md | ns | ns | 0.00005 | 0.02565 |
| 5 | PPP1CA | 0.00109 | 0.00349 | ns | ns | 0.01239 | ns | ns | ns | 0.00471 | 0.00024 |
| 5 | SLC25A5 | 0.00191 | ns1 | 0.01349 | ns | 0.02281 | ns1 | ns | ns | 0.00039 | 0.00382 |
| 4 | BTG2 | 0.00001 | 0.00543 | ns1 | ns1, 2 | 0.00020 | ns1 | ns1 | 0.01687 | ns | ns |
| 4 | C4B | 0.00061 | ns1 | ns | 0.01467 | ns1 | ns | ns | ns | 0.00401 | 0.02725 |
| 4 | VEGFA | 0.00020 | ns1, 2 | 0.01384 | ns | 0.00294 | ns1 | ns | ns | ns2 | 0.04090 |
| 4 | SFRS5 | 0.00077 | ns1 | ns | ns2 | 0.01678 | ns | ns | 0.03148 | 0.02179 | ns |
| 4 | ENO1 | 0.00008 | 0.01194 | md | ns | 0.01341 | md | ns | ns | 0.02788 | ns |
| 4 | GATA3 | 0.00060 | ns1 | 0.02185 | ns | 0.02143 | ns1 | ns | ns | 0.01549 | ns |
| 4 | TMED9 | 0.00415 | 0.00025 | ns | ns | ns1 | ns | ns | 0.00002 | ns | 0.00192 |
| 4 | POR | 0.01509 | ns1 | ns | ns | 0.00959 | ns | ns | ns | 0.00001 | 0.03871 |
| 3 | PSMB3 | 0.00177 | ns1, 2 | ns | ns | ns1 | ns | ns | ns | 0.03941 | 0.00096 |
| 3 | CYC1 | 0.00091 | 0.00148 | ns | ns | ns1, 2 | ns2 | ns2 | ns | 0.00105 | ns2 |
| 3 | XIST | 0.00027 | md | md | ns2 | md | md | ns | ns | 0.00913 | 0.00941 |
| 3 | C17orf37 | 0.00272 | md | md | md | md | md | md | md | 0.03881 | 0.00073 |
| 2 | FABP5 | 0.00058 | ns | ns1, 2 | ns | ns | ns1 | ns | 0.01527 | ns2 | ns |
| 2 | PSMB7 | 0.00059 | ns1, 2 | ns | ns | ns1 | ns | 0.04657 | ns | ns | ns |
| 2 | TUBA4A | 0.00153 | ns | ns1, 2 | ns | ns | ns1 | ns | ns | 0.01089 | ns |
| 2 | DDT | 0.00124 | 0.04183 | ns | ns | ns1 | ns | ns | ns | ns2 | ns2 |
| 2 | CHCHD2 | 0.00035 | md | md | ns | md | md | ns | ns | 0.00016 | ns2 |
| 2 | RAB10 | 0.00218 | ns2 | ns | md | ns | ns | md | md | 0.00065 | ns |
| 2 | RPN1 | 0.00161 | ns | md | ns | ns | md | ns | ns | ns2 | 0.00856 |
| 2 | ETFB | 0.00175 | ns | 0.01651 | ns | ns | ns1 | ns | ns | ns | ns2 |
| 2 | SURF4 | 0.00018 | md | md | md | md | md | md | md | 0.00051 | ns |
| 2 | TNFSF13 | 0.00013 | ns | ns | ns | ns | ns | ns | 0.00979 | ns | ns |
| 2 | PPA1 | 0.00204 | ns | ns | ns | ns | ns | ns | ns | ns | 0.00778 |
| 2 | BCL2A1 | 0.00215 | ns | ns | ns | ns | ns | ns | ns | 0.01237 | ns |
| 2 | PSMA5 | 0.00673 | ns | ns | ns | ns | ns | ns | ns | ns | 0.00589 |
| 1 | C1orf64 | 0.00087 | md | md | md | md | md | md | md | ns | ns2 |
| 1 | TUBB6 | 0.00028 | md | md | ns | md | md | ns | ns | ns2 | ns |
| 1 | C3orf37 | 0.00291 | ns | ns | ns | ns | ns | ns | ns | ns2 | ns |
| 1 | TOE1 | 0.00296 | md | md | ns | md | md | ns | ns | ns | ns |
| 1 | RHOC | 0.00047 | ns | ns | ns | ns | ns | ns | ns | ns | ns |
| **N° of genes with:** P < 0.05 | | 38 | 10 | 4 | 1 | 13 | 0 | 4 | 9 | 22 | 17 |
| P < 0.10 | | 38 | 14 | 6 | 5 | 14 | 1 | 6 | 9 | 28 | 22 |

TC: training cohort; VC: validation cohort 1 to 3; EC: exploratory cohort 1 to 6; ns: non significant (P > 0.05); md: missing data; 1: P < 0.05 in VCi∪ECi: i = 1, 2 or 3; 2: 0.05 < P < 0.10

A large majority (86%) of the 38 genes showed a significant p-value in at least one external subcohort or cohort (table VIII). Genes reaching or exceeding 50% of significance in the 10 groups are: *UBE2C, TACC3, IL6ST, TUBB2C, TUBB3, PGK1, PPP1CA* and *SLC25A5*. These results confirmed that the vast majority of the genes retained in our 38-GES are biologically relevant and linked to breast cancer.

## CONCLUSION:

The above results show that the 38-GES:

1) had predictive value in node-positive patients treated by chemotherapy independently of known prognostic parameters such as age, SBR grade, tumoral size, ER or number of positive nodes (alone or combined into NPI and Adjuvant!); 2) could improve classification of NPI and Adjuvant!, especially for intermediate-risk patients; 3) confirmed its performance in node-positive independent cohort; 4) had also prognostic value in node-negative patients.

Without being bound by theory, it is proposed that the information contained in our 38-GES relates to a common molecular background, in node-positive and node-negative patients, linked to bad outcome prediction, and that this 38-GES is dominated by bad prognosis-associated genes which are also predictive of general chemotherapy sensitivity.

**EXAMPLE 4: COMPARISON OF 70-GES CLASSIFICATION AND 38-GES CLASSIFICATION**

The aim of this study was to assess whether the 38-GES kept its prognostic ability when adjusted to the 70-GES reference signature (Amsterdam signature [Mammaprint® test], VAN'T VEER et al Nature 2002, cited above) in multivariate Cox models.

The study was conducted on different populations of cancer patients:

1) Node-positive population (MR N+);
2) Node-negative population (MR N-);
3) Population with mixed node status and for who only undefined relapse event is known (UR N mixed);
4) Pool of populations (1) and (2) (MR N+ and N-);
5) Pool of populations (1) (2) and (3) (All).

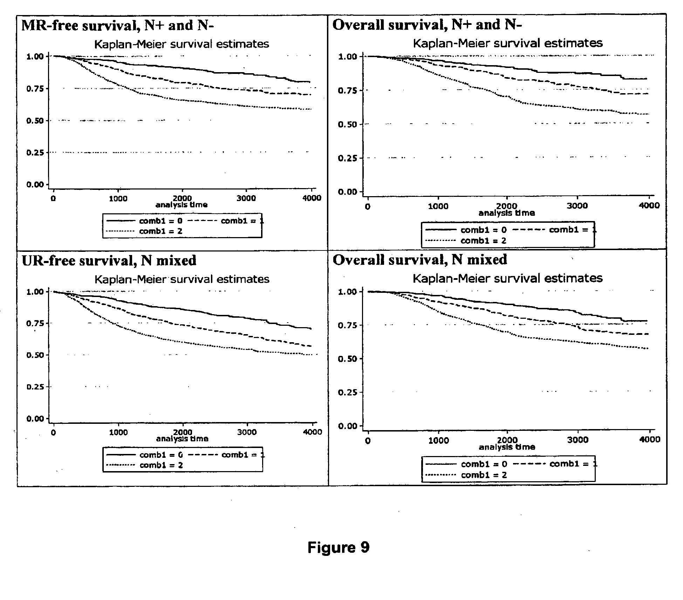

The results are shown in Table IX below, and in Figure 9.

Table IX

| | Relapse (MR or UR) | | | Death | | |
|---|---|---|---|---|---|---|
| | HR | HR 95% CI | P | HR | HR 95% CI | P |
| MR N+ (n=917) | | | | | | |
| 70-GES | 1.24 | 0.90-1.72 | 0.1902 | 1.37 | 0.91-2.07 | 0.1320 |
| 38-GES | 2.34 | 1.81-3.04 | <0.0001 | 2.13 | 1.54-2.96 | <0.0001 |
| | | | | | | |
| MR N- (n=1573) | | | | | | |
| 70-GES | 2.47 | 1.82-3.37 | <0.0001 | 2.68 | 1.67-4.30 | <0.0001 |
| 38-GES | 1.27 | 1.01-1.59 | 0.0439 | 1.29 | 0.88-1.88 | 0.1890 |
| | | | | | | |
| UR N mixed (n=951) | | | | | | |
| 70-GES | 1.34 | 1.02-1.77 | 0.0384 | 0.99 | 0.68-1.46 | 0.988 |
| 38-GES | 1.37 | 1.10-1.69 | 0.0042 | 1.41 | 1.05-1.89 | 0.023 |
| | | | | | | |
| MR N+ et N- (n=2490) | | | | | | |
| 70-GES | 1.80 | 1.40-1.97 | <0.0001 | 1.83 | 1.34-2.51 | <0.0001 |
| 38-GES | 1.66 | 1.44-2.25 | <0.0001 | 1.75 | 1.36-2.24 | <0.0001 |
| | | | | | | |
| All (n=3441) | | | | | | |
| 70-GES | 1.58 | 1.35-1.85 | <0.0001 | 1.50 | 1.18-1.91 | 0.001 |
| 38-GES | 1.51 | 1.33-1.70 | <0.0001 | 1.63 | 1.35-1.97 | <0.0001 |
| MR: metastatic relapse; UR: undefined relapse; HR: hazard ratio; CI: confidence interval; N: node | | | | | | |

Figure 9 shows Kaplan-Meier survival curves of 38-GES and 70-GES combined classification in the different populations (MR: metastatic relapse; N: node; UR: undefined relapse). The three groups (comb1) are: 0 = 38-GES and 70-GES "good prognosis" groups; 1 = 38-GES or 70-GES "bad prognosis" group; 2 = 38-GES and 70-GES "bad prognosis" groups

From the results of Table IX and Figure 9, the following conclusions can be drawn:

1) Concerning node-positive population (1), prognostic information is mostly supported by 38-GES both for metastatic relapse and death. This is not completely surprising as the 38-GES was established based on node-positive patients). 2) Concerning node-negative population (2), prognostic information is mainly supported by 70-GES both for metastatic relapse and death. This is also not completely surprising as the 70-GES was established based on node-negative patients. One can however notice that in the case of metastatic relapse, the 38-GES remains significant when adjusted to the 70-GES. 3) Concerning population with mixed node status and for whom only undefined event is known (3), the 38-GES has a better prognostic informativity than the 70-GES. More specifically, only 38-GES supports prognostic information for death prediction, whereas 70-GES adds significant prognostic information for relapse prediction. 4) When pooling all populations (1+2 or 1+2+3), both signatures provide very significant and complementary prognostic information (see Figure 9).

**Claims**

1.   A method for evaluating the risk of metastatic relapse in a breast cancer patient, wherein said method comprises:

- measuring the level of expression of each of the following genes: BTG2, UBE2C, VEGFA, SLC25A5, FABP5, ENO1, PSMB7, TUBB6, TACC3, SURF4, RHOC, CHCHD2, SFRS5, TNFSF13, DDT, C1orf64, RAB10, XIST, TUBA4A, PPA1, C4B, TUBB2C, C3orf37, GATA3, PGK1, PPP1CA, BCL2A1, IL6ST, PSMB3, RPN1, ETFB, CYC1, POR, TUBB3, TMED9, TOE1, C17orf37, PSMA, in a sample of breast tumoral tissue obtained from said patient, and
- comparing the level of expression of each of said genes with the mean level of expression of the same gene measured in samples of breast tumoral tissue from a reference group of patients with breast cancer, wherein an overexpression of UBE2C, VEGFA, SLC25A5, FABP5, ENO1, PSMB7, TUBB6, TACC3, SURF4, RHOC, CHCHD2, DDT, RAB10, TUBA4A, PPA1, TUBB2C, C3orf37, PGK1, PPP1CA, BCL2A1, PSMB3, RPN1, ETFB, CYC1, POR, TUBB3, TMED9, TOE1, C17orf37, PSMA5, and an underexpression of BTG2, SFRS5, TNFSF13, C1orf64, XIST, C4B, GATA3, IL6ST, are correlated with a higher probability of metastatic relapse.

2.   The method of claim 1, wherein the level of expression of said genes is measured by determination of their level of transcription, using a DNA array.

3.   The method of claim 1, wherein the level of expression of said genes is measured by determination of their level of transcription, using quantitative RT-PCR.

4.   The method of any of claims 1 to 3, wherein a risk score (Pi) for a given patient is calculated according to the following equation:

$$\mathrm{Pi} = \frac{1}{30}\sum_{i=1}^{30} Gi - \frac{1}{8}\sum_{j=1}^{8} Gj$$

wherein $Gi$ represents overexpressed gene standardized expression value and $Gj$ represents underexpressed gene standardized expression value, and wherein overexpressed genes are *UBE2C, VEGFA, SLC25A5, FABP5, ENO1, PSMB7, TUBB6, TACC3, SURF4, RHOC, CHCHD2, DDT, RAB10, TUBA4A, PPA1, TUBB2C, C3orf37, PGK1, PPP1CA, BCL2A1, PSMB3, RPN1, ETFB, CYC1, POR, TUBB3, TMED9, TOE1, C17orf37, PSMA5*, and underexpressed genes are *BTG2, SFRS5, TNFSF13, C1orf64, XIST, C4B, GATA3, IL6ST.*

5.   A kit for evaluating the probability of metastatic relapse in a breast cancer patient, **characterized in that** it consists of a combination of reagents for measuring the level of expression of each of the 38 genes listed in claim 1.

6.   A kit of claim 5, **characterized in that** it contains a DNA array comprising for each of the 38 genes listed in claim 1, at least one nucleic acid probe specific of said gene.

7.  A kit of claim 5, **characterized in that** it is a PCR kit, containing, for each of the 38 genes listed in claim 1, at least one pair of PCR primers specific of said gene.

**Patentansprüche**

1.  Verfahren zur Bewertung des Risikos von Metastaserückfall in einem Brustkrebspatienten, wobei das Verfahren umfasst:

    - Messen des Expressionslevels von jedem der folgenden Gene: BTG2, UBE2C, VEGFA, SLC25A5, FABP5, ENO1, PSMB7, TUBB6, TACC3, SURF4, RHOC, CHCHD2, SFRS5, TNFSF13, DDT, C1orf64, RAB10, XIST, TUBA4A, PPA1, C4B, TUBB2C, C3orf37, GATA3, PGK1, PPP1CA, BCL2A1, IL6ST, PSMB3, RPN1, ETFB, CYC1, POR, TUBB3, TMED9, TOE1, C17orf37, PSMA in einer Probe von Brusttumorgewebe, welche von dem Patienten erhalten wurde, und
    - Vergleichen des Expressionslevels von jedem der Gene mit dem mittleren Expressionslevel des gleichen Gens, welches in Proben von Brusttumorgewebe von einer Referenzgruppe von Patienten mit Brustkrebs gemessen worden ist, wobei eine Überexpression von UBE2C, VEGFA, SLC25A5, FABP5, ENO1, PSMB7, TUBB6, TACC3, SURF4, RHOC, CHCHD2, DDT, RAB10, TUBA4A, PPA1, TUBB2C, C3orf37, PGK1, PPP1CA, BCL2A1, PSMB3, RPN1, ETFB, CYC1, POR, TUBB3, TMED9, TOE1, C17orf37, PSMA5 und eine Unterexpression von BTG2, SFRS5, TNFSF13, C1orf64, XIST, C4B, GATA3, IL6ST, mit einer höheren Wahrscheinlichkeit von Metastaserückfall korelliert sind.

2.  Verfahren gemäß Anspruch 1, wobei das Expressionslevel der Gene durch Bestimmung ihres Transkriptionslevels mit Hilfe eines DNA-Arrays gemessen wird.

3.  Verfahren gemäß Anspruch 1, wobei das Expressionslevel der Gene durch Bestimmung ihres Transkriptionslevels mit Hilfe quantitativer RT-PCR gemessen wird.

4.  Verfahren gemäß einem der Ansprüche 1 bis 3, wobei eine Risikopunktzahl (Pi) für einen gegebenen Patienten gemäß der folgenden Gleichung berechnet wird:

$$Pi = \frac{1}{30}\sum_{i=1}^{30} Gi - \frac{1}{8}\sum_{j=1}^{8} Gj$$

    wobei *Gi* einen standardisierten Expressionswert eines überexprimierten Gens darstellt, und *Gj* einen standardisierten Expressionswert eines unterexprimierten Gens darstellt, und wobei überexprimierte Gene *UBE2C, VEGFA, SLC25A5, FABP5, ENO1, PSMB7, TUBB6, TACC3, SURF4, RHOC, CHCHD2, DDT, RAB10, TUBA4A, PPA1, TUBB2C,* C3orf37, *PGK1, PPP1CA, BCL2A1, PSMB3, RPN1, ETFB, CYC1, POR, TUBB3, TMED9, TOE1, C17orf37, PSMA5* sind, und unterexprimierte Gene *BTG2, SFRS5, TNFSF13, C1orf64, XIST, C4B, GATA3, IL6ST* sind.

5.  Kit zur Bewertung der Wahrscheinlichkeit von Metastaserückfall in einem Brustkrebspatienten, **dadurch gekennzeichnet, dass** es aus einer Kombination von Reagenzien zur Messung des Expressionslevels von jedem der in Anspruch 1 aufgelisteten 38 Gene besteht.

6.  Kit gemäß Anspruch 5, **dadurch gekennzeichnet, dass** es einen DNA-Array enthält, welcher für jedes der in Anspruch 1 aufgelisteten 38 Gene mindestens eine Nucleinsäuresonde umfasst, welche für das Gen spezifisch ist.

7.  Kit gemäß Anspruch 5, **dadurch gekennzeichnet, dass** es ein PCR-Kit ist, welches für jedes der in Anspruch 1 aufgelisteten 38 Gene mindestens ein Paar von PCR-Primern enthält, welches für das Gen spezifisch ist.

**Revendications**

1. Procédé pour évaluer le risque d'une rechute métastatique chez une patiente souffrant d'un cancer mammaire, dans lequel ledit procédé comprend :

   - la mesure du niveau d'expression de chacun des gènes suivants : BTG2, UBE2C, VEGFA, SLC25A5, FABP5, ENO1, PSMB7, TUBB6, TACC3, SURF4, RHOC, CHCHD2, SFRS5, TNFSF13, DDT, C1orf64, RAB10, XIST, TUBA4A, PPA1, C4B, TUBB2C, C3orf37, GATA3, PGK1, PPP1CA, BCL2A1, IL6ST, PSMB3, RPN1, ETFB, CYC1, POR, TUBB3, TMED9, TOE1, C17orf37, PSMA, dans un échantillon de tissu cancéreux mammaire prélevé chez ladite patiente, et

   - la comparaison du niveau d'expression de chacun desdits gènes au niveau moyen d'expression du même gène mesuré dans des échantillons de tissu cancéreux mammaire provenant d'un groupe de référence de patientes souffrant d'un cancer mammaire, dans lequel une surexpression de UBE2C, VEGFA, SLC25A5, FABP5, ENO1, PSMB7, TUBB6, TACC3, SURF4, RHOC, CHCHD2, DDT, RAB10, TUBA4A, PPA1, TUBB2C, C3orf37, PGK1, PPP1CA, BCL2A1, PSMB3, RPN1, ETFB, CYC1, POR, TUBB3, TMED9, TOE1, C17orf37, PSMA5, et une sous-expression de BTG2, SFRS5, TNFSF13, C1orf64, XIST, C4B, GATA3, IL6ST, sont corrélées à une plus grande probabilité de rechute métastatique.

2. Procédé selon la revendication 1, dans lequel le niveau d'expression desdits gènes est mesuré par la détermination de leur niveau de transcription, au moyen d'une puce à ADN.

3. Procédé selon la revendication 1, dans lequel le niveau d'expression desdits gènes est mesuré par la détermination de leur niveau de transcription, au moyen d'une RT-PCR quantitative.

4. Procédé selon l'une quelconque des revendications 1 à 3, dans lequel un score de risque (Pi) pour une patiente donnée est calculé selon l'équation suivante :

$$\text{Pi} \quad = \frac{1}{30} \sum_{i=1}^{30} Gi - \frac{1}{8} \sum_{j=1}^{8} Gj$$

dans laquelle *Gi* représente une valeur d'expression standardisée d'un gène surexprimé et *Gj* représente une valeur d'expression standardisée d'un gène sous-exprimé, et dans lequel les gènes surexprimés sont *UBE2C, VEGFA, SLC25A5, FABP5, ENO1, PSMB7, TUBB6, TACC3, SURF4, RHOC, CHCHD2, DDT, RAB10, TUBA4A, PPA1, TUBB2C, C3orf37, PGK1, PPP1CA, BCL2A1, PSMB3, RPN1, ETFB, CYC1, POR, TUBB3, TMED9, TOE1, C17orf37, PSMA5,* et les gènes sous-exprimés sont *BTG2, SFRS5, TNFSF13, C1orf64, XIST, C4B, GATA3, IL6ST.*

5. Kit pour évaluer la probabilité d'une rechute métastatique chez une patiente souffrant d'un cancer mammaire, caractérisé en qu'il est constitué d'une combinaison de réactifs pour mesurer le niveau d'expression de chacun des 38 gènes listés dans la revendication 1.

6. Kit selon la revendication 5, caractérisé en qu'il contient une puce à ADN comprenant, pour chacun des 38 gènes listés dans la revendication 1, au moins une sonde d'acides nucléiques spécifique dudit gène.

7. Kit selon la revendication 5, caractérisé en que c'est un kit PCR contenant, pour chacun des 38 gènes listés dans la revendication 1, au moins une paire d'amorces PCR spécifiques dudit gène.

**Figure 1**

**Figure 2**

**Figure 3**

**Figure 4**

**A**

## Metastasis-free survival according to 38-GES & NPI

Patients at risk

| | | | | | | |
|---|---|---|---|---|---|---|
| — NPI 1 | 26 | 26 | 26 | 24 | 14 | 0 |
| — NPI=2/GES- | 93 | 93 | 90 | 82 | 32 | 1 |
| -- NPI=2 | 136 | 133 | 124 | 112 | 45 | 1 |
| —NPI=2/GES+ | 43 | 40 | 34 | 30 | 13 | 0 |
| — NPI=3 | 206 | 176 | 138 | 114 | 44 | 0 |

**B**

## Metastasis-free survival according to 38-GES & Adjuvant!

Patients at risk

| | | | | | | |
|---|---|---|---|---|---|---|
| — AOL>80 | 46 | 46 | 46 | 42 | 22 | 0 |
| — AOL 60-80/GES- | 64 | 64 | 61 | 55 | 22 | 1 |
| -- AOL 60-80 | 90 | 89 | 82 | 72 | 29 | 1 |
| —AOL 60-80/GES+ | 26 | 25 | 21 | 17 | 7 | 0 |
| — AOL<60 | 278 | 244 | 202 | 176 | 68 | 0 |

**Figure 5**

**Figure 6**

**Figure 7**

**A**

Metastasis-free survival according to 38-GES

Probability of MFS — 1, .75, .5, .25, 0
HR=1.93 (1.44-2.58)- p<0.0001
Years — 0 2 4 6 8 10 12

Patients at risk
low-risk 284 270 238 193 124 53 20
high-risk 348 281 231 178 138 71 39

**B**

38-GES score, for MR at 5 years

Sensitivity — 1, .75, .5, .25, 0
AUC = 0.646
1 - specificity — 0.00 0.25 0.50 0.75 1.00

**C**

38-GES and 5-year distant relapse: %, (95% CI)

| | |
|---|---|
| Sensitivity | 0.70 (0.67-0.74) |
| Specificity | 0.52 (0.48-0.56) |
| Positive predictive value | 0.37 (0.33-0.40) |
| Negative predictive value | 0.81 (0.78-0.85) |

**D**

Event-free survival according to 38-GES

Probability of MFS — 1, .75, .5, .25, 0
HR=2.64 (1.85-3.75)-p<0.0001
Years — 0 2 4 6 8 10 12

Patients at risk
low-risk 240 218 202 175 121 80 0
high-risk 168 120 97 82 51 37 7

**E**

38-GES score, for UE at 5 years

Sensitivity — 1, .75, .5, .25, 0
AUC = 0.684
1 - specificity — 0.00 0.25 0.50 0.75 1.00

**F**

38-GES and 5-year cancer event: %, (95% CI)

| | |
|---|---|
| Sensitivity | 0.64 (0.59-0.69) |
| Specificity | 0.68 (0.63-0.73) |
| Positive predictive value | 0.43 (0.38-0.48) |
| Negative predictive value | 0.83 (0.80-0.87) |

**Figure 8**

**Figure 9**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **GALEA et al.** *Breast Cancer Res Treat,* 1992, vol. 22, 207-219
- **RAVDIN et al.** *J Clin Oncol,* 2001, vol. 19, 980-991
- **PEROU et al.** *Nature,* 2000, vol. 406, 747-52
- **SORLIE et al.** *Proc Natl Acad Sci USA,* 2001, vol. 98, 10869-74
- **VAN'T VEER et al.** *Nature,* 2002, vol. 415, 530-35
- **VAN DE VIJVER et al.** *N Engl J Med,* 2002, vol. 347, 1999-2009
- **WANG et al.** *The Lancet,* 2005, vol. 365, 671-79
- **PAIK et al.** *N Engl J Med,* 2004, vol. 351, 2817-26
- **MA et al.** *Cancer Cell,* 2004, vol. 5, 607-16
- **FREEMAN et al.** *Biotechniques,* 24 May 1999, vol. 26, 112-22
- **BUSTIN ; MUELLER.** *Clin Sci (Lond),* 2005, vol. 109, 365-79
- **BERTUCCI et al.** *Hum. Mol. Genet.,* 1999, vol. 8, 1715-22
- **CHURCHILL.** *Nat Genet,* 2002, vol. 32, 490-5
- **HELLER.** *Annu Rev Biomed Eng,* 2002, vol. 4, 129-53
- **RAMASWAMY ; GOLUB.** *J Clin Oncol,* 2002, vol. 20, 1932-41
- **AFFARA.** *Brief Funct Genomic Proteomic,* 2003, vol. 2, 7-20
- **COPLAND et al.** *Recent Prog Horm Res,* 2003, vol. 58, 25-53
- **SCHROEDER et al.** *BMC Molecular Biology,* 2006, vol. 7, 3
- **LI ; STORMO.** *Bioinformatics,* 2001, vol. 17, 1067-76

- **EMRICH et al.** *Nucleic Acids Res,* 2003, vol. 31, 3746-50
- **ROUILLARD et al.** *Nucl. Acids Res.,* 2003, vol. 31, 3057-62
- **BINAS.** *Biotechniques,* 2000, vol. 29, 988-90
- **ROZEN ; SKALETSKY.** *Methods Mol Biol,* 2000, vol. 132, 365-86
- **GORELENKOV et al.** *Biotechniques,* 2001, vol. 31, 1326-30
- **LEE et al.** *Appl Bioinformatics,* 2006, vol. 5, 99-109
- **YAMADA et al.** *Nucleic Acids Res,* 2006, vol. 34, W665-9
- **ROCHÉ et al.** *J Clin Oncol,* 2006, vol. 24, 5664-71
- **CAMPONE et al.** *Breast Cancer Res Treat,* 2007, vol. 109, 491-501
- **SOTIRIOU et al.** *Proc Natl Acad Sci USA,* 2003, vol. 100, 10393-98
- **SOTIRIOU et al.** *J Natl Cancer Inst,* 2006, vol. 98, 262-72
- **PAWITAN et al.** *Breast Cancer Res,* 2005, vol. 7, R953-R964
- **IVSHINA et al.** *Cancer Res,* 2006, vol. 66, 10292-301
- **CHIRGWIN et al.** *Biochemistry,* 1979, vol. 18, 5294-99
- **MAGRANGEAS et al.** *Blood,* 2003, vol. 101, 4998-5006
- **DECAUX et al.** *J Clin Oncol,* 2008, 26
- **DUPUY ; SIMON.** *J Natl Cancer Inst,* 2007, vol. 99, 147-157
- **VAN'T VEER et al.** *Nature,* 2002